# EUROPEAN PATENT SPECIFICATION

(11) **EP 4 384 299 B1**
(45) Date of publication and mention of the grant of the patent: **29.07.2026**
(21) Application number: 22856732.7
(22) Date of filing: 05.08.2022
(51) Int. Cl.: B01D 19/02

(54) **APPARATUS AND METHOD FOR REMOVING BUBBLES FROM FLUID SAMPLE WITH SLIDABLE FILTER**
VORRICHTUNG UND VERFAHREN ZUR ENTFERNUNG VON BLASEN AUS EINER FLÜSSIGKEITSPROBE MIT VERSCHIEBBAREM FILTER
APPAREIL ET PROCÉDÉ POUR ÉLIMINER DES BULLES D'UN ÉCHANTILLON DE FLUIDE AVEC UN FILTRE COULISSANT

(30) Priority: 12.08.2021 US 202163232365 P
(43) Date of publication of application: 19.06.2024
(73) Proprietor: Siemens Healthcare Diagnostics Inc., Tarrytown, NY 10591 (US)
(72) Inventor: COX, Janine, Stoughton, Massachusetts 02072 (US); DESILVA, Justin, Mendon, Massachusetts 01756 (US)
(74) Representative: Schweitzer, Klaus
(86) International application number: PCT/US2022/074577
(87) International publication number: WO 2023/019085

(56) References cited:
- CA-A1- 3 122 260
- GB-A- 2 043 478
- US-A- 4 571 244
- US-A- 4 572 210
- US-A- 4 998 926
- US-A1- 2005 065 454
- US-A1- 2006 111 667
- US-A1- 2011 108 158
- US-B2- 7 500 569
- US-B2- 7 537 571
- US-B2- 7 691 332

## Description

### BACKGROUND

Blood sampling is a common health care procedure typically used in hospital and laboratory settings to determine the physiological and biochemical condition of a patient. Blood sampling is essential to the diagnosis and treatment of patients suspected of a wide variety of disorders. Blood samples are analyzed by fluid testing devices, such as blood analyzers, to detect clinically significant variations in blood components, e.g., plasma, red blood cells, white blood cells, and platelets, or other characteristics, such as blood gas conditions. Analysis of a blood sample for blood gas conditions provides information regarding the amount of oxygen and carbon dioxide in the blood, and may also be used to determine the pH of a blood sample. Imbalances in the oxygen, carbon dioxide, or pH levels of a blood sample may indicate particular pathological conditions or stage of disease progression.

Blood samples are typically collected using blood collection syringes having hypodermic needles, or vacuum tubes coupled to a needle assembly. However, blood collection syringes are prone to have bubbles of air or other gases within the syringe. The bubbles in the barrel and tip of the syringe may interfere with the analysis of blood samples. Regarding blood gas analysis, bubbles trapped in a syringe may cause a blood analyzer to produce erroneous results. To obtain accurate results, blood samples need to be mixed thoroughly and all air must be expelled before mixing. Bubbles are typically expelled by tapping the sides of the syringe to force the air or gas to the top of the syringe. Once the bubbles have reached the top of the syringe, a piece of gauze or tissue is put over the top to force the gas (and some blood) out of the syringe. This method poses not only a biohazard risk because the blood sample is freely flowing out of the top of the syringe, but also an exposure risk, as the tip of the syringe is still open to ambient air. A blood analyzer analyzing a blood sample collected in this manner risks producing erroneous results.

Further, blood analyzers often may not permit direct sample input via a syringe or vacuum tube. In that case, the syringe and/or vacuum tube is merely an intermediate container for the blood sample, and at least a portion of the blood sample must be removed and transferred to a secondary sample container capable of being accommodated by the blood analyzer. Transferring the blood sample to a secondary sample container presents its own exposure risks and may increase the likelihood of obtaining erroneous results.

It is known in the prior art (CA 3 122 260 A1) a lateral flow testing device having a first fluid reservoir, a second fluid reservoir defined by a lower portion, a third fluid reservoir defined by at least a portion of an upper portion, and a lateral flow membrane. The second fluid reservoir and the third fluid reservoir are separated by the lateral flow membrane. As a fluid sample is transferred from the second fluid reservoir to the third fluid reservoir, the fluid sample passes through the lateral flow membrane and the fluid sample is separated into at least two constituent parts, e.g., blood cells remain in the second fluid reservoir or are captured within the lateral flow membrane and plasma passes through the lateral flow membrane and into the third fluid reservoir.

US 2006/111667 A1 discloses a syringe assembly having a syringe device, which comprises a syringe cavity, and a cap. The cap has a body portion. The body portion has one end and a distal end to which a hydrophobic filter membrane is ultrasonically welded, thereby covering an aperture at the distal end. The hydrophobic filter membrane allows the passage of gas but not liquid.

US 4 998 926 A discloses an air-permeable liquid-impermeable element which is attached to a flange so that gases passing through an aperture must pass through the element. The air-permeable liquid-impermeable element is porous and capable of developing sufficiently high surface tension under liquid contact so that it serves as a barrier against liquid passage therethrough during the normal use of the container assembly.

The disclosure of GB 2 043 478 A1 describes a filter device comprising a filter membrane through which the parenteral solution passes (preferably "hydrophilic"). That is, when wetted by solution, it is permeable to liquid but not gas. To vent the gas separated by the hydrophilic filter membrane, a cap has an off-centre vent. A "hydro-phobic" filter membrane, which is liquid-repellent and gas permeable, is sealed against the underside of the cap below the vent to permit gas to vent but to prevent fluid from escaping through the vent.

An example of a degassing apparatus that has a slidable membrane member is disclosed in US4572210A.

A need exists for an apparatus and method for removing bubbles from a fluid sample. It is to such an apparatus and method that the inventive concepts disclosed and claim herein are directed.

### SUMMARY OF THE INVENTIVE CONCEPTS

The inventive concepts disclosed and claimed herein generally relate to an apparatus for removing bubbles from a fluid sample having a liquid portion and a gas portion. The apparatus includes a barrel and a filter member. The barrel has a first end, a second end, a sidewall, and an inner surface. The sidewall extends between the first end and the second end of the barrel, and the inner surface of the barrel defines an internal chamber. The first end of the barrel has an inlet opening and the second end has an outlet opening. The filter member is disposed within the internal chamber so the filter member defines an inlet side and an outlet side of the internal chamber. The filter member is positioned between the first end and the second end of the filter member. The filter member has at least one gas-permeable, liquid-impermeable membrane to permit at least a portion of the gas portion of the fluid sample to pass across the filter member from the inlet side to the outlet side of the internal chamber. The at least one gas-permeable, liquid-impermeable membrane provides a fluid-tight seal cross the filter member to prevent the liquid portion of the fluid sample from passing from the inlet side to the outlet side as the fluid sample is passed into the internal chamber via the inlet opening to separate at least a portion of the gas portion from the liquid portion of the fluid sample. The filter member is pierceable by a sample probe so the probe may be passed through the filter member from the outlet side to the inlet side to withdraw the liquid portion of the fluid sample from the inlet side of the internal chamber. The filter member is slidably disposed in the internal chamber of the barrel. The inventive apparatus is defined in claim 1 and the associated method is defined in claim 10.

### BRIEF DESCRIPTION OF THE DRAWINGS

To assist those of ordinary skill in the relevant art in making and using the inventive concepts disclosed herein, reference is made to the appended drawings and schematics, which are not intended to be drawn to scale, and in which like reference numerals are intended to refer to the same or similar elements for consistency. For purposes of clarity, not every component may be labeled in every drawing. Certain features and certain views of the figures may be shown exaggerated and not to scale or in schematic in the interest of clarity and conciseness. In the drawings:
FIG. 1 is a side perspective view of an exemplary embodiment of an apparatus for removing bubbles according to the inventive concepts disclosed herein shown coupled to a sample receiving assembly.
FIG. 2A is a longitudinal, cross-sectional view of the apparatus of FIG. 1 coupled to a collection syringe illustrating positioning of a filter member and a plunger assembly prior to removal of bubbles from the fluid sample.
FIG. 2B is a longitudinal, cross-sectional view of the apparatus of FIG. 1 coupled to a collection syringe illustrating positioning of the filter member and plunger assembly following removal of bubbles from the fluid sample.
FIG. 2C is a longitudinal, cross-sectional view of the apparatus of FIG. 1 coupled to a collection syringe illustrating the insertion of a probe into the apparatus following removal of bubbles from of the fluid sample.
FIG. 3A is a longitudinal, cross-sectional view of the apparatus of FIG. 1 illustrating a position of the filter member prior to removal of bubbles from the fluid sample.
FIG. 3B is a is a longitudinal, cross-sectional view of the apparatus of FIG. 1 illustrating a position of the filter member following removal of bubbles from the fluid sample.
FIG. 3C is a longitudinal, cross-sectional view of the apparatus of FIG. 1 illustrating the insertion of the probe into the apparatus following removal of bubbles from the fluid sample.
FIG. 4 is a perspective view of the apparatus.
FIG. 5 is an exploded, cross-sectional view of the apparatus.
FIG. 6A is a perspective view of an exemplary embodiment of the filter member according to the inventive concepts disclosed herein.
FIG. 6B is a cross-sectional view taken along line 6B-6B of FIG. 6A.
FIG. 7 is a perspective view of another embodiment of a filter member according to the inventive concepts disclosed herein.
FIG. 8A is a perspective view of another embodiment of a filter member according to the inventive concepts disclosed herein.
FIG. 8B is a cross-sectional view taken along line 8B-8B of FIG. 8A

### DETAILED DESCRIPTION OF EXEMPLARY EMBODIMENTS

Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. The language used herein is intended to be given the broadest possible scope and meaning; and the embodiments are meant to be exemplary-not exhaustive. Also, it is to be understood that the phraseology and terminology employed herein is for the purpose of description. The present invention is defined by the appended independent claims. Specific embodiments are defined in the dependent claims.

Unless otherwise defined, scientific and technical terms used in connection with the presently disclosed and claimed inventive concept(s) shall have the meanings commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses and chemical analyses.

All the articles, compositions and/or methods disclosed and claimed herein can be made and executed without undue experimentation, given the present disclosure.

As utilized under the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one."

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the inherent variation of error for the device, the method being employed to determine the value, or the variation that exists among the study subjects.

The use of the term "at least one" will be understood to include one as well as any quantity more than one, including 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y, and Z" will be understood to include X alone, Y alone, and Z alone, as well as any combination of X, Y, and Z.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

As used herein, the term "sample" and variations thereof is intended to include biological tissues, biological fluids, chemical fluids, chemical substances, suspensions, solutions, slurries, mixtures, agglomerations, tinctures, slides, powders, or other preparations of biological tissues or fluids, synthetic analogs to biological tissues or fluids, bacterial cells (prokaryotic or eukaryotic), viruses, single-celled organisms, lysed biological cells, fixed biological cells, fixed biological tissues, cell cultures, tissue cultures, genetically engineered cells and tissues, genetically engineered organisms, and combinations thereof, for example.

In the following detailed description of embodiments of the inventive concept, numerous specific details are set forth in order to provide a more thorough understanding of the inventive concept.

Finally, as used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Described herein, and shown in the accompanying figures, are several embodiments of apparatus of the presently claimed and disclosed inventive concepts which may be used in association with collection syringes and fluid sample analyzers for removing bubbles of air or other gases from a fluid sample for analysis by a fluid sample analyzer. The fluid sample is generally from a biological source. A "fluid" refers to any substance that has no fixed shape and yields easily to external pressure.

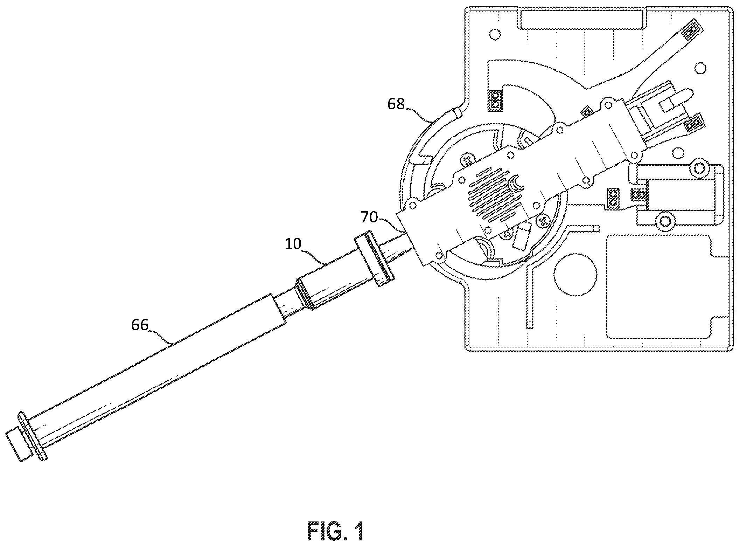

Referring now to the drawings, and more particularly to FIGS. 1-5, shown therein is an exemplary embodiment of an apparatus 10 for removing bubbles from a fluid sample constructed in accordance with the inventive concepts disclosed and claimed herein. The apparatus 10 includes a barrel 12, a nozzle cap 14, and a filter member 16.

The barrel 12 includes a first end 18, a second end 20, a sidewall 22, and an inner surface 24. The barrel 12 may be of any suitable size and shape, and formed of any suitable material, such as plastics such as polycarbonate, polystyrene, polyacrylates, and polyurethane, or medical-grade polymers. The sidewall 22 of the barrel 12 extends between the first end 18 and the second end 20 of the barrel 12. The inner surface 24 of the barrel 12 defines an internal chamber 26. The first end 18 has an inlet opening 28 and the second end 20 has an outlet opening 30.

The internal chamber 26 may be of any suitable size and shape to contain a fluid sample 32. The fluid sample 32 may be, for example, blood, serum, plasma, or other bodily fluids. The fluid sample 32 may contain a gas portion and a liquid portion. The gas portion of the fluid sample 32 may be, for example, air or other gases. A portion of the gas portion may form bubbles in the fluid sample

The inlet opening 28 and the outlet opening 30 may have a cross-section of any suitable geometry, including circular, oval, square, or rectangular. The inlet opening 28 and the outlet opening 30 may be molded or cut into the barrel 12, or otherwise pre-fabricated. The inlet opening 28 may be formed to prevent coagulation as the fluid sample 32 is passed into the internal chamber 26 via the inlet opening 28.

The outlet opening 30 is provided with a nozzle cap 14. The nozzle cap 14 includes an annular wall 36 and a tubular portion 38 having a bore 40 extending therethrough. The bore 40 may have a cross-section of any suitable geometry, including circular, oval, square, or rectangular. The bore 40 may be sized to have a diameter adapted to slidably axially receive a sample probe. The base of the tubular portion 38 flares outwardly and merges with the annular wall 36 at a rim 42, the annular wall 36 tapers downwardly to form an inverted frusto-conical section. The nozzle cap 14 is releasably coupled to the outlet opening 30 such that the bore 40 is aligned with the outlet opening 30 to permit fluid communication with the internal chamber 26.

The filter member 16 is disposed within the internal chamber 26 so the filter member 16 defines an inlet side 44 and an outlet side 46 of the internal chamber 26. The filter member 16 is positionable between the first end 18 and the second end 20 of the barrel 12. The filter member 16 includes at least one gas-permeable, liquid-impermeable membrane 48. The filter member 16 may be any suitable shape and size to sealingly engage the inner surface 24 of the barrel 12. The filter member 16 may be formed of any suitable material, such as a rubber, an elastomer, a polyolefin-based resin, a fluorine-based resin, or a polyester-based resin. The elastomer may include, for example, a polyvinyl chloride-based elastomer, a polyolefin-based elastomer, a styrene-based elastomer, a polyester-based elastomer, a polyamide-based elastomer, a polyurethane-based elastomer, or a mixture thereof.

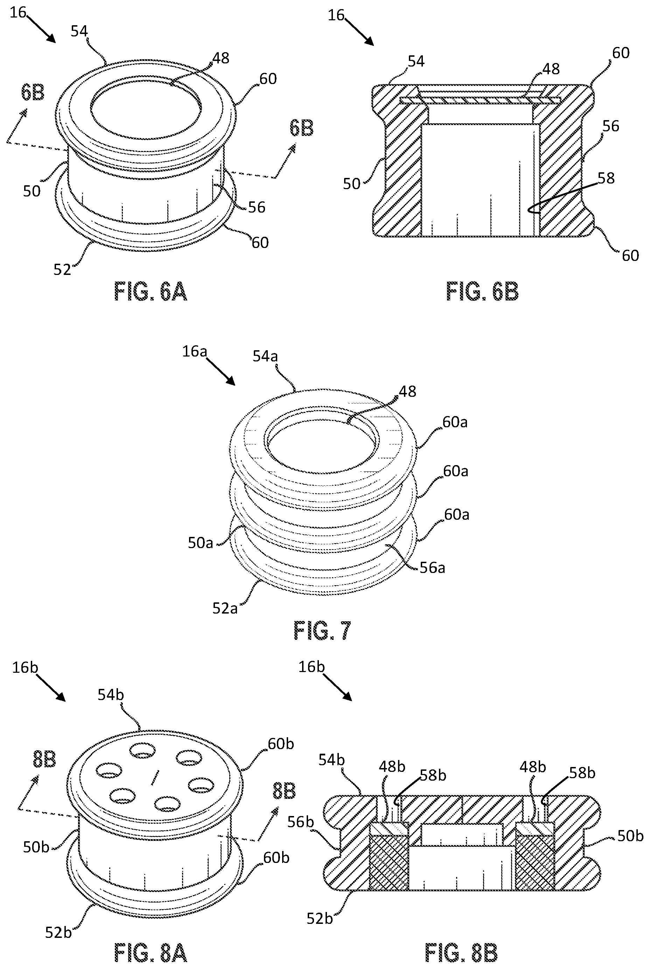

Referring now to FIGS. 6A and 6B, shown therein is a perspective and cross-sectional view, respectively, of an exemplary embodiment of the filter member 16. The filter member 16 comprises a body 50 having a first end 52, a second end 54, and a sidewall 56 extending from the first end 52 to the second end 54. The sidewall 56 of the body 50 defines a passageway 58 extending through the body 50 from the first end 52 to the second end 54. The sidewall 56 of the body 50 may have at least two annular projections 60 extending radially outwardly in slidable, sealing contact with the inner surface 24 of the barrel 12. As shown in FIGS. 6A and 6B, the body 50 may have two annular projections 60 spaced apart from each other. The annular projections 60 may include convex or concave features.

The filter member 16 further includes a gas-permeable, liquid-impermeable membrane 48 which extends across the entirety of the passageway 58. In one embodiment, the gas-permeable, liquid-impermeable membrane 48 is secured to the body 50 adjacent the second end 54 of the body 50, as shown in FIG. 6B.

Referring now to FIG. 7, shown therein is a perspective view of another embodiment of a filter member 16a constructed in accordance with the inventive concepts disclosed and claimed herein. Similar to previously described embodiments, the filter member 16a comprises a body 50a having a first end 52a, a second end 54a, and a sidewall 56a extending from the first end 52a to the second end 54a. The sidewall 56a of the body 50a defines a passageway 58a (not shown) extending through the body 50a from the first end 52a to the second end 54a. As in FIGS. 6A and 6B of the previously described embodiment, the body 50a may have at least two annular projections 60b extending radially outwardly in slidable, sealing contact with the inner surface 24 of the barrel 12. As shown in FIG. 7, the body 50a may have three annular projections 60a spaced apart from each other. The filter member 16a further includes the gas-permeable, liquid-impermeable membrane 48 which extends across the entirety of the passageway 58a. In this embodiment, the gas-permeable, liquid-impermeable membrane 48 is secured to the body 50a adjacent the second end 54a of the body.

Referring now to FIGS. 8A and 8B, shown therein is a perspective and cross-sectional view, respectively, of another embodiment of a filter member 16b. The filter member 16b comprises a body 50b having a first end 52b, a second end 54b, and a sidewall 56b extending from the first end 52b to the second end 54b. Similar to previously described embodiments, the body 50b may have at least two annular projections 50b extending radially outwardly in slidable, sealing contact with the inner surface 24 of the barrel 12. The sidewall 56b of the body 50b defines a plurality of passageways 58b extending through the body 50b from the first end 52b to the second end 54b. In that embodiment, the plurality of passageways 58b may be in a parallel relationship to one another, as shown in FIG. 8B. Further, the filter member 16b may include a plurality of gas-permeable, liquid-impermeable membranes 48b with at least one of the gas-permeable, liquid-impermeable membranes 48b extending across each of the plurality of passageways 58b of the body 50b. The filter member 16b may further include a plurality of porous filter material 62 positioned between the first end 52b of the body 50b and each of the plurality of gas-permeable, liquid-impermeable membranes 48b to prevent solid particulate from contacting the plurality of gas-permeable, liquid-impermeable membranes 48b, as shown in FIG. 8B.

The at least one gas-permeable, liquid-impermeable membrane 48 may be formed of any suitable material, such as polytetrafluoroethylene, polyvinylidene fluoride, polyvinylchloride, polyolefins like polypropylene, polyethylene, polymethylpentene, polyamides, polysulfones, polyetheretherketones, polycarbonates, and combinations including any of these. In one embodiment, the gas-permeable, liquid-impermeable membrane 48 is formed from a material comprising at least one of polytetrafluorethylene, polypropylene, and polyethylene. The at least one gas-permeable, liquid-impermeable membrane 48 may have a thickness suitable for allowing puncture upon application of a mechanical force. The at least one gas-permeable, liquid-impermeable membrane 48 may permit at least a portion of the gas portion of the fluid sample 32, which forms bubbles, to pass across the filter member 16 from the inlet side 44 to the outlet side 46 of the internal chamber 26. The at least one gas-permeable, liquid-impermeable membrane 48 also provides a fluid-tight seal across the filter member 16 to prevent the liquid portion of the fluid sample 32 from passing from inlet side 44 to the outlet side 46 as the fluid sample 32 is passed into the internal chamber 26 via the inlet opening 28 to separate at least a portion of the gas portion from the liquid portion of the fluid sample 32. The filer member 16 is pierceable so a sample probe 72 may be passed through the filter member 16 from the outlet side 46 to the inlet side 44 to withdraw the liquid portion of the fluid sample 32 from the inlet side 44 of the internal chamber 26.

As shown in FIG. 1, the apparatus 10 may be used in association with a collection syringe 66 and a fluid sample analyzer 68 to remove bubbles from the fluid sample 32 having a liquid portion and a gas portion. Although, FIG. 1 shows the apparatus 10 associated with the collection syringe 66 and the fluid sample analyzer 68, those skilled in the art will understand and appreciate that the apparatus 10 may independently be associated with collection devices other than the collection syringe 66, such as, for example, a vacuum tube, and medical devices other than the fluid sample analyzer 68. The fluid sample analyzer 68 may be any suitable fluid testing device, such as microfluidic devices, blood gas analyzers, hematology analyzers, urine chemistry analyzers, and the like.

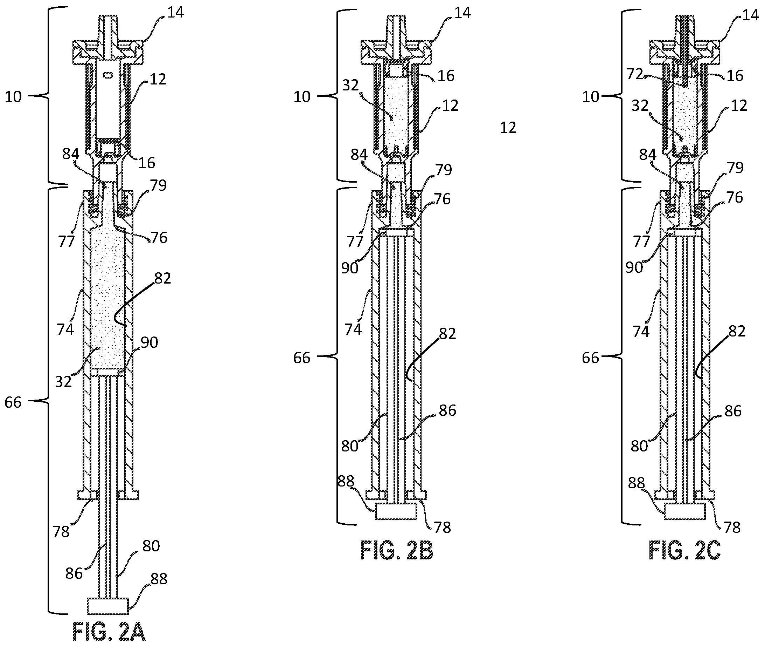

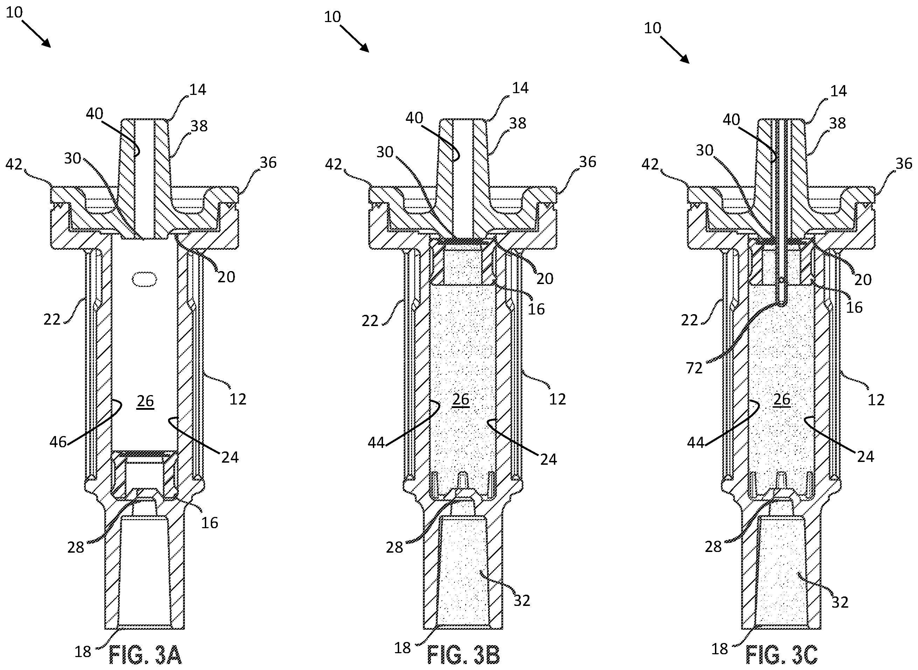

The fluid sample analyzer 68 includes a sample input port 70 and a sample probe 72. The sample probe 72 may be axially slidable relative to the sample input port 70. The sample input port 70 may be sized to receive and detachably secure at least a portion of the nozzle cap 14, as shown in FIG. 1. The sample probe 72 may be extended to pass through the filter member 16 from the outlet side 46 of the internal chamber 26 to the inlet side 44 to withdraw at least a portion of the fluid sample 32 into the fluid sample analyzer 68 from the inlet side 44 of the internal chamber 26, as shown in FIGS. 2C and 3C. The sample probe 72 may be of a length compatible with sampling from the inlet side 44 of the internal chamber 26.

The collection syringe 66 includes a syringe body 74 having a front end 76, a rear end 78, and a plunger 80. The syringe body 74 defines a reservoir 82 within which the fluid sample 32 may be contained and later expelled via a dispensing opening 84 positioned at the front end 76 of the syringe body 74. The rear end 78 of the syringe body 74 may be open and provided with a body flange 85 to facilitate the collection and expulsion of the fluid sample 32. The syringe body 74 may be any suitable size or shape for collection of fluid samples, such as, for example, a cylindrical shape. The syringe body 74 may also include a collar 77 formed concentrically with the dispensing opening 84 into a cylindrical shape to surround the dispending opening 84. The collar 77 may include an inner peripheral surface in which a threaded engagement portion 79 is formed for engaging the apparatus 10. The syringe body 74 may be constructed of any suitable material, such as glass or plastic. The syringe body 74 may have an outer diameter adapted to coaxially slide within inlet opening 28 of the apparatus 10.

The plunger 80 may include a shaft 86 that terminates at one end in a plunger flange 88 to facilitate the collection and expulsion of the fluid sample 32. The shaft 86 may, for example, have a cylindrical shape or a columnar shape, and may have a cross-section of a polygonal shape, such as a square, pentagonal, hexagonal, or cruciform shape. The plunger 80 may further include a plunger seal 90 secured to the shaft 86 opposite the plunger flange 88. The plunger 80 may be removably disposed within the syringe body 74 and may be selectively movable within the reservoir 82. The plunger seal 90 has a diameter that permits the plunger seal 90 to create a fluid-tight seal when positioned within the reservoir 82 such that the liquid sample 32 may not move past the plunger seal 90. Further, the plunger seal 90 prevents ambient air from moving from the rear end 78 of the syringe body 74 in a direction past the plunger seal 90. The plunger 80 may be axially displaced relative to the syringe body 74. Movement of the plunger 80 from the rear end 78 to the front end 76 of the syringe body 74 may cause at least a portion of the fluid sample 32 to be expelled from the reservoir 82 and introduced into the inlet opening 28 of the apparatus 10 via the dispensing opening 84. The plunger 80 may be constructed of any suitable polymeric material known in the art.

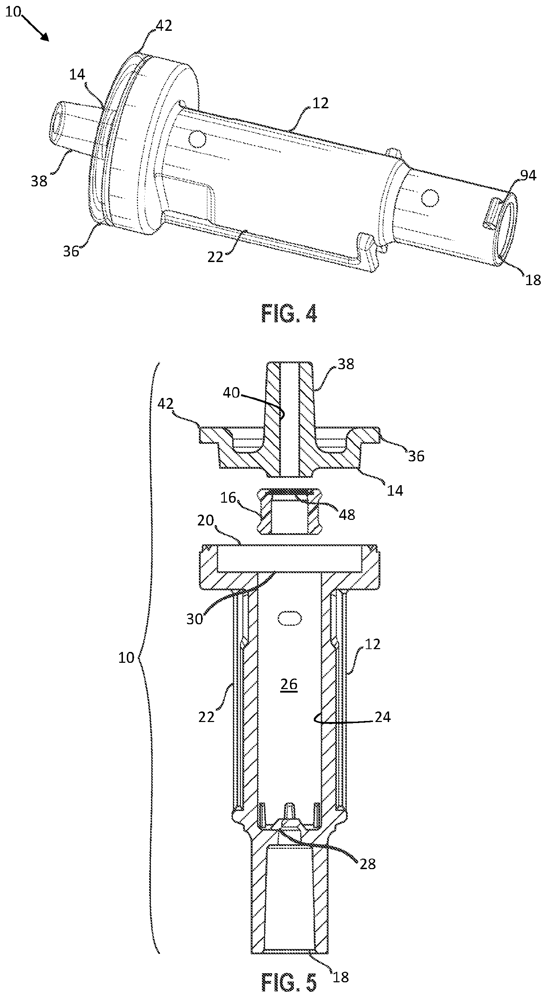

To remove the gas portion (i.e., bubbles) of the fluid sample 32 from the liquid portion, the collection syringe 66, containing a volume of the fluid sample 32 within the reservoir 82, is releasably attached to the inlet opening 28 of the barrel 12, as shown in FIG. 2A. As shown in FIGS. 2A-2C, the front end 76 of the syringe body 74 may be releasably connected to the apparatus 10 by way of the threaded engagement portion 79. As shown in FIG. 4, the barrel 12 may include a barrel connection portion 94. In one embodiment, the threaded engagement portion 79 of the syringe body 74 is a male luer connection and the barrel connection portion 94 is a female luer connector, although other suitable connectors may be utilized. As shown in FIGS. 2A-2C, the threaded engagement portion 79 may detachably engage the barrel connection portion 94 such that significant relative movement between the collection syringe 66 and the apparatus 10 is prevented, and fluid communication between the reservoir 82 and the inlet opening 28 of the apparatus 10 is permitted.

The collection syringe 66 and the apparatus 10 are positioned in an upright orientation with the apparatus 10 above the collection syringe 66 and the bubbles in the fluid sample rise to the top of the fluid sample. The plunger 80 of the collection syringe 66 is displaced axially along the reservoir 82 a distance from the rear end 78 towards the front end 76 of the syringe body 74, as shown in FIG. 2A. Movement of the plunger 80 within the reservoir 82 causes at least a portion of the gas portion (i.e., bubbles) of the fluid sample 32 to be expelled from the reservoir 82 and into the internal chamber 26 of the apparatus 10 via the inlet opening 28, passing through the filter member 16. The gas portion of the fluid sample 32 passes through the filter member 16, and is then ultimately expelled from the internal chamber 26 of the apparatus 10. Once, at least a portion of the gas portion of the fluid sample 32 has been displaced from the reservoir 82, the plunger 80 experiences an initial resistive force.

Upon application of sufficient force to overcome the initial resistive force, the plunger 80 is advanced further into the reservoir 82 towards the front end 76 of the syringe body 74, as shown in FIG. 2B, thereby increasing the internal pressure of the reservoir 82. As the internal pressure of the reservoir 82 increases, it produces a force sufficient to cause at least a portion of the liquid portion of the fluid sample 32 to be expelled from the reservoir 82 and into the internal chamber 26 of the barrel 12 via the inlet opening 28. The fluid sample 32 entering the internal chamber 26 may cause the filter member 16 to be displaced axially along the internal chamber 26 towards the second end 20 of the barrel 12, as shown in FIGS. 2B and 3B. The filter member 16 may be displaced such that it becomes disposed adjacent the outlet opening 30. This arrangement prevents ambient air from entering the internal chamber 26 and prevents the fluid sample 32 from exiting the internal chamber 26 via the outlet opening 30. In some embodiments, the plunger 80 may be partially extended into the reservoir 82 so less than all of the fluid sample 32 is transferred from the reservoir 82 into the internal chamber 26.

Once the liquid portion of the fluid sample 32 has been expelled from the reservoir 82 and is contained within the internal chamber 26 of the apparatus 10, the sample probe 72 of the fluid sample analyzer 68 may be extended from the sample input port 70 and passed through the filter member 16 to withdraw the liquid portion of the fluid sample 32 from the inlet side 44 of the internal chamber 26, as shown in FIGS. 2C and 3C. In one embodiment, the sample probe 72 pierces the gas-permeable, liquid-impermeable membrane 48 of the filter member 16 to gain fluid access to the inlet side 44 of the internal chamber 26.

## Claims

1. An apparatus (10) for removing bubbles from a fluid sample (32) having a liquid portion and a gas portion, comprising:
a barrel (12) having a first end (18), a second end (20), a sidewall (22) extending between the first end and the second end, and an inner surface (24) defining an internal chamber (26), the first end (18) having an inlet opening (28) and the second end having an outlet opening (30); and
a filter member (16) disposed within the internal chamber (26) so the filter member defines an inlet side (44) and an outlet side (46) of the internal chamber and so the filter member (16) is positionable between the first end (18) and the second end of the barrel (20), the filter member (16) having at least one gas-permeable, liquid-impermeable membrane (48) to permit at least a portion of the gas portion of the fluid sample (32) to pass across the filter member from the inlet side (44) to the outlet side (46) of the internal chamber (26) and to provide a fluid-tight seal across the filter member to prevent the liquid portion of the fluid sample from passing from the inlet side (44) to the outlet side (46) as the fluid sample (32) is passed into the internal chamber (26) via the inlet opening (28) to separate at least a portion of the gas portion from the liquid portion of the fluid sample;
wherein the gas-permeable, liquid-impermeable membrane (48) of the filter member (16) is pierceable by a sample probe (72) so the probe may be passed through the filter member (16) from the outlet side (46) to the inlet side (44) to withdraw the liquid portion of the fluid sample (32) from the inlet side (44) of the internal chamber (26),
wherein the filter member (16) further comprises a body (50) having a first end (52), a second end (54), and a sidewall (56) extending from the first end to the second end, the sidewall (56) of the body defining a passageway (58) extending through the body (50) from the first end to the second end, and wherein the gas-permeable, liquid-impermeable membrane (48) extends across an entirety of the passageway, and wherein the outlet opening (30) is provided with a nozzle cap (14) ) including an annular wall (36) and a tubular portion (38) having a bore (40) extending therethrough, wherein the nozzle cap (14) is releasably coupled to the outlet opening (30) such that the bore (40) is aligned with the outlet opening (30) to permit fluid communication with the internal chamber (26), wherein the filter member (16) is slidably disposed in the internal chamber (26) of the barrel.

2. An apparatus for removing bubbles from a fluid sample (32) having a liquid portion and a gas portion according to claim 1, wherein the apparatus is connected to a liquid sample analyzer (68) having the sample probe (72),
wherein the second end of the barrel (12) being connected to the liquid sample analyzer (68); and
wherein the filter member (16) is pierceable by the sample probe so the sample probe may be passed through the outlet opening (30) of the barrel (12) and through the filter member (16) from the outlet side (46) to the inlet side (44) to draw the liquid portion of the fluid sample (32) into the liquid sample analyzer from the inlet side (44) of the internal chamber (26).

3. The apparatus of claim 1, wherein the gas-permeable, liquid-impermeable membrane (48) is secured to the body (50) adjacent the second end of the body (50).

4. The apparatus of claim 1, wherein the sidewall of the body (50) has at least two annular projections extending radially outwardly in slidable, sealing contact with the inner surface (24) of the barrel (12).

5. The apparatus of claim 1 or 2, wherein the gas-permeable, liquid-impermeable membrane (48) is formed from a material comprising at least one of polytetraflyorethylene, polypropylene, and polyethylene.

6. The apparatus of claim 1, wherein the body (50) has a plurality of passageways extending through the body from the first end (52) to the second end (54), and wherein the filter member (16) further has a plurality of gas-permeable, liquid impermeable membranes with at least one of the gas-permeable, liquid impermeable membranes extending across each of the passageways of the body (50).

7. The apparatus of claim 6, wherein the filter member (16) further comprises a plurality of porous filter material positioned between the first end of the body (50) and each of the gas-permeable, liquid impermeable membranes to prevent solid particulate from contacting the gas-permeable, liquid impermeable membranes.

8. The apparatus of claim 6, wherein the plurality of gas-permeable, liquid-impermeable membranes (48) are secured to the body (50) adjacent the second end (54) of the body.

9. The apparatus of claim 6, wherein the passageways are in a parallel relationship to one another.

10. A method of removing bubbles from a fluid sample (32) having a liquid portion and a gas portion using an apparatus (10) as claimed in claim 1, the method comprising:
receiving a flow of the fluid sample (32) from an inlet opening (28) of a barrel (12) having a first end (18), a second end (20), a sidewall (22) extending between the first end and the second end, and an inner surface (24) defining an internal chamber (26), the first end (18) having the inlet opening (28) and the second end having an outlet opening (30);
passing the fluid sample into the internal chamber via the inlet opening (28);
causing the fluid sample (32) to contact a filter member (16) disposed within the internal chamber (26), the filter member (16) defining an inlet side (44) and an outlet side (46) of the internal chamber, the filter member (16) having a gas-permeable, liquid-impermeable membrane (48);
separating at least a portion of the gas portion of the liquid sample from the liquid portion of the fluid sample by causing the fluid sample (32) to contact the gas-permeable, liquid-impermeable membrane (48) so that at least a portion of the gas portion of the fluid sample (32) passes across the filter member (16) from the inlet side to the outlet side (46) of the internal chamber (26) and so the liquid portion of the fluid sample (32) is prevented from passing from the inlet side (44) to the outlet side (46);
collecting at least a portion of the liquid portion of the fluid sample (32) from the inlet side of the internal chamber (26).

11. The method of claim 10, wherein at least a portion of the liquid portion of the fluid sample (32) is collected from the inlet side (44) of the internal chamber by piercing the filter member so a probe may be passed through the filter member (16) from the outlet side (46) to the inlet side (44) to withdraw the liquid portion of the fluid sample from the inlet side (44) of the internal chamber (26).

12. The method of claim 10, wherein the filter member (16) further comprises a filter element interposed between the gas-permeable, liquid-impermeable membrane and the inlet side (44) of the internal chamber (26), the at least one filter element having an inlet port and an outlet port, the inlet port in fluid communication with the inlet side (44) of the internal chamber (26), the outlet port in fluid communication with the gas-permeable, liquid-impermeable membrane to permit at least a portion of the fluid sample (32) to pass across the at least one filter element and to the gas-permeable, liquid-impermeable membrane.

13. The method of claim 10, wherein the gas-permeable, liquid-impermeable membrane is a plurality of gas-permeable, liquid-impermeable membranes.

## Patentansprüche

1. Vorrichtung (10) zur Entfernung von Blasen aus einer Fluidprobe (32) mit einem Flüssigkeitsanteil und einem Gasanteil, Folgendes umfassend:
einen Zylinder (12) mit einem ersten Ende (18), einem zweiten Ende (20), einer Seitenwand (22), die sich zwischen dem ersten Ende und dem zweiten Ende erstreckt, und einer Innenfläche (24), die eine Innenkammer (26) definiert, wobei das erste Ende (18) eine Einlassöffnung (28) aufweist und das zweite Ende eine Auslassöffnung (30) aufweist; und
ein Filterelement (16), das innerhalb der Innenkammer (26) angeordnet ist, so dass das Filterelement eine Einlassseite (44) und eine Auslassseite (46) der Innenkammer definiert und so das Filterelement (16) zwischen dem ersten Ende (18) und dem zweiten Ende des Zylinders (20) positionierbar ist, wobei das Filterelement (16) mindestens eine gasdurchlässige, flüssigkeitsundurchlässige Membran (48) aufweist, um zu ermöglichen, dass mindestens ein Teil des Gasanteils der Fluidprobe (32) von der Einlassseite (44) zu der Auslassseite (46) der Innenkammer (26) durch das Filterelement hindurchgeht, und um eine flüssigkeitsdichte Abdichtung über das Filterelement bereitzustellen, um zu verhindern, dass der Flüssigkeitsanteil der Fluidprobe von der Einlassseite (44) zu der Auslassseite (46) gelangt, wenn die Fluidprobe (32) über die Einlassöffnung (28) in die Innenkammer (26) geleitet wird, um mindestens einen Teil des Gasanteils von dem Flüssigkeitsanteil der Fluidprobe zu trennen;
wobei die gasdurchlässige, flüssigkeitsundurchlässige Membran (48) des Filterelements (16) durch eine Probensonde (72) durchstechbar ist, so dass die Sonde durch das Filterelement (16) von der Auslassseite (46) zu der Einlassseite (44) geführt werden kann, um den Flüssigkeitsanteil der Fluidprobe (32) von der Einlassseite (44) der Innenkammer (26) zu entnehmen,
wobei das Filterelement (16) ferner einen Körper (50) mit einem ersten Ende (52), einem zweiten Ende (54) und einer Seitenwand (56), die sich von dem ersten Ende zu dem zweiten Ende erstreckt, umfasst, wobei die Seitenwand (56) des Körpers einen Durchgang (58) definiert, der sich durch den Körper (50) von dem ersten Ende zu dem zweiten Ende erstreckt, und wobei sich die gasdurchlässige, flüssigkeitsundurchlässige Membran (48) über eine Gesamtheit des Durchgangs erstreckt, und wobei die Auslassöffnung (30) mit einer Düsenkappe (14) versehen ist, die eine ringförmige Wand (36) und einen röhrenförmigen Abschnitt (38) mit einer Bohrung (40) beinhaltet, die sich durch diese erstreckt, wobei die Düsenkappe (14) lösbar mit der Auslassöffnung (30) gekoppelt ist, so dass die Bohrung (40) mit der Auslassöffnung (30) ausgerichtet ist, um eine Fluidverbindung mit der Innenkammer (26) zu ermöglichen, wobei das Filterelement (16) verschiebbar in der Innenkammer (26) des Zylinders angeordnet ist.

2. Vorrichtung zur Entfernung von Blasen aus einer Fluidprobe (32) mit einem Flüssigkeitsanteil und einem Gasanteil nach Anspruch 1, wobei die Vorrichtung mit einem Flüssigkeitsprobenanalysator (68) mit der Probensonde (72) verbunden ist,
wobei das zweite Ende des Zylinders (12) mit dem Flüssigkeitsprobenanalysator (68) verbunden ist; und
wobei das Filterelement (16) durch die Probensonde durchstechbar ist, so dass die Probensonde durch die Auslassöffnung (30) des Zylinders (12) und durch das Filterelement (16) von der Auslassseite (46) zu der Einlassseite (44) geführt werden kann, um den Flüssigkeitsanteil der Fluidprobe (32) von der Einlassseite (44) der Innenkammer (26) in den Flüssigkeitsprobenanalysator zu ziehen.

3. Vorrichtung nach Anspruch 1, wobei die gasdurchlässige, flüssigkeitsundurchlässige Membran (48) an dem Körper (50) angrenzend an das zweite Ende des Körpers (50) befestigt ist.

4. Vorrichtung nach Anspruch 1, wobei die Seitenwand des Körpers (50) mindestens zwei ringförmige Vorsprünge aufweist, die sich radial nach außen in verschiebbarem, abdichtendem Kontakt mit der Innenfläche (24) des Zylinders (12) erstrecken.

5. Vorrichtung nach Anspruch 1 oder 2, wobei die gasdurchlässige, flüssigkeitsundurchlässige Membran (48) aus einem Material gebildet ist, das Polytetrafluorethylen, Polypropylen und/oder Polyethylen umfasst.

6. Vorrichtung nach Anspruch 1, wobei der Körper (50) mehrere Durchgänge aufweist, die sich von dem ersten Ende (52) zu dem zweiten Ende (54) durch den Körper erstrecken, und wobei das Filterelement (16) ferner mehrere gasdurchlässige, flüssigkeitsundurchlässige Membranen aufweist, wobei sich mindestens eine der gasdurchlässigen, flüssigkeitsundurchlässigen Membranen über jeden der Durchgänge des Körpers (50) erstreckt.

7. Vorrichtung nach Anspruch 6, wobei das Filterelement (16) ferner mehrere poröse Filtermaterialien umfasst, die zwischen dem ersten Ende des Körpers (50) und jeder der gasdurchlässigen, flüssigkeitsundurchlässigen Membranen positioniert sind, um zu verhindern, dass feste Partikel mit den gasdurchlässigen, flüssigkeitsundurchlässigen Membranen in Kontakt kommen.

8. Vorrichtung nach Anspruch 6, wobei die mehreren gasdurchlässigen, flüssigkeitsundurchlässigen Membranen (48) am Körper (50) angrenzend an das zweite Ende (54) des Körpers befestigt sind.

9. Vorrichtung nach Anspruch 6, wobei die Durchgänge in einer parallelen Beziehung zueinander stehen.

10. Verfahren zur Entfernung von Blasen aus einer Fluidprobe (32) mit einem Flüssigkeitsanteil und einem Gasanteil unter Verwendung einer Vorrichtung (10) nach Anspruch 1, wobei das Verfahren Folgendes umfasst:
Empfangen eines Stroms der Fluidprobe (32) von einer Einlassöffnung (28) eines Zylinders (12) mit einem ersten Ende (18), einem zweiten Ende (20), einer Seitenwand (22), die sich zwischen dem ersten Ende und dem zweiten Ende erstreckt, und einer Innenfläche (24), die eine Innenkammer (26) definiert, wobei das erste Ende (18) die Einlassöffnung (28) aufweist und das zweite Ende eine Auslassöffnung (30) aufweist;
Leiten der Fluidprobe über die Einlassöffnung (28) in die Innenkammer;
Bewirken, dass die Fluidprobe (32) ein Filterelement (16) berührt, das innerhalb der Innenkammer (26) angeordnet ist, wobei das Filterelement (16) eine Einlassseite (44) und eine Auslassseite (46) der Innenkammer definiert, wobei das Filterelement (16) eine gasdurchlässige, flüssigkeitsundurchlässige Membran (48) aufweist;
Trennen mindestens eines Teils des Gasanteils der Fluidprobe von dem Flüssigkeitsanteil der Fluidprobe, indem bewirkt wird, dass die 'Fluidprobe (32) mit der gasdurchlässigen, flüssigkeitsundurchlässigen Membran (48) in Kontakt kommt, so dass mindestens ein Teil des Gasanteils der Fluidprobe (32) von der Einlassseite zu der Auslassseite (46) der Innenkammer (26) durch das Filterelement (16) hindurchgeht, sodass verhindert wird, dass der Flüssigkeitsanteil der Fluidprobe (32) von der Einlassseite (44) zu der Auslassseite (46) gelangt;
Sammeln mindestens eines Teils des Flüssigkeitsanteils der Fluidprobe (32) von der Einlassseite der Innenkammer (26).

11. Verfahren nach Anspruch 10, wobei mindestens ein Teil des Flüssigkeitsanteils der Fluidprobe (32) von der Einlassseite (44) der Innenkammer gesammelt wird, indem das Filterelement durchstochen wird, so dass eine Sonde durch das Filterelement (16) von der Auslassseite (46) zu der Einlassseite (44) geführt werden kann, um den Flüssigkeitsanteil der Fluidprobe von der Einlassseite (44) der Innenkammer (26) abzuziehen.

12. Verfahren nach Anspruch 10, wobei das Filterelement (16) ferner ein Filterelement umfasst, das zwischen der gasdurchlässigen, flüssigkeitsundurchlässigen Membran und der Einlassseite (44) der Innenkammer (26) angeordnet ist, wobei das mindestens eine Filterelement eine Einlassöffnung und eine Auslassöffnung aufweist, wobei die Einlassöffnung in Fluidverbindung mit der Einlassseite (44) der Innenkammer (26) steht, wobei die Auslassöffnung in Fluidverbindung mit der gasdurchlässigen, flüssigkeitsundurchlässigen Membran steht, damit zumindest ein Teil der Fluidprobe (32) über das mindestens eine Filterelement und zu der gasdurchlässigen, flüssigkeitsundurchlässigen Membran gelangen kann.

13. Verfahren nach Anspruch 10, wobei die gasdurchlässige, flüssigkeitsundurchlässige Membran mehrere gasdurchlässige, flüssigkeitsundurchlässige Membranen ist.

## Revendications

1. Appareil (10) pour enlever des bulles d'un échantillon de fluide (32) ayant une partie liquide et une partie gazeuse, comprenant :
un cylindre (12) ayant une première extrémité (18), une seconde extrémité (20), une paroi latérale (22) s'étendant entre la première extrémité et la seconde extrémité, et une surface intérieure (24) définissant une chambre interne (26), la première extrémité (18) ayant une ouverture d'entrée (28) et la seconde extrémité ayant une ouverture de sortie (30) ; et
un élément filtrant (16) disposé à l'intérieur de la chambre interne (26) de telle sorte que l'élément filtrant définit un côté entrée (44) et un côté sortie (46) de la chambre interne et que l'élément filtrant (16) peut être positionné entre la première extrémité (18) et la seconde extrémité du cylindre (20), l'élément filtrant (16) ayant au moins une membrane (48) perméable aux gaz et imperméable aux liquides permettant à au moins une partie de la partie gazeuse de l'échantillon de fluide (32) de passer à travers l'élément filtrant du côté entrée (44) au côté sortie (46) de la chambre interne (26) et fournissant un joint étanche aux fluides à travers l'élément filtrant afin d'empêcher la partie liquide de l'échantillon de fluide de passer du côté entrée (44) au côté sortie (46) lorsque l'échantillon de fluide (32) passe dans la chambre interne (26) par le biais de l'ouverture d'entrée (28) pour séparer au moins une partie de la partie gazeuse de la partie liquide de l'échantillon de fluide ;
dans lequel la membrane (48) perméable aux gaz et imperméable aux liquides de l'élément filtrant (16) peut être percée par une sonde d'échantillon (72) de telle sorte que la sonde peut être passée à travers l'élément filtrant (16) du côté sortie (46) au côté entrée (44) pour retirer la partie liquide de l'échantillon de fluide (32) du côté entrée (44) de la chambre interne (26),
dans lequel l'élément filtrant (16) comprend en outre un corps (50) ayant une première extrémité (52), une seconde extrémité (54), et une paroi latérale (56) s'étendant de la première extrémité à la seconde extrémité, la paroi latérale (56) du corps définissant un passage (58) s'étendant à travers le corps (50) de la première extrémité à la seconde extrémité, et dans lequel la membrane (48) perméable aux gaz et imperméable aux liquides s'étend à travers la totalité du passage, et dans lequel l'ouverture de sortie (30) est pourvue d'un bouchon de buse (14) comprenant une paroi annulaire (36) et une partie tubulaire (38) ayant un alésage (40) traversant, dans lequel le bouchon de buse (14) est accouplé de manière amovible à l'ouverture de sortie (30) de telle sorte que l'alésage (40) est aligné avec l'ouverture de sortie (30) pour permettre une communication fluidique avec la chambre interne (26), l'élément filtrant (16) étant disposé de manière coulissante dans la chambre interne (26) du cylindre.

2. Appareil pour enlever des bulles d'un échantillon de fluide (32) ayant une partie liquide et une partie gazeuse selon la revendication 1, dans lequel l'appareil est relié à un analyseur d'échantillon de liquide (68) comportant la sonde d'échantillon (72),
dans lequel la seconde extrémité du cylindre (12) est reliée à l'analyseur d'échantillon de liquide (68) ; et
dans lequel l'élément filtrant (16) peut être percé par la sonde d'échantillon de telle sorte que la sonde d'échantillon peut passer à travers l'ouverture de sortie (30) du cylindre (12) et à travers l'élément filtrant (16) du côté sortie (46) au côté entrée (44) pour retirer la partie liquide de l'échantillon de fluide (32) dans l'analyseur d'échantillon de liquide du côté entrée (44) de la chambre interne (26).

3. Appareil selon la revendication 1, dans lequel la membrane perméable aux gaz et imperméable aux liquides (48) est fixée au corps (50) de façon adjacente à la seconde extrémité du corps (50).

4. Appareil selon la revendication 1, dans lequel la paroi latérale du corps (50) comporte au moins deux saillies annulaires s'étendant radialement vers l'extérieur en contact coulissant et étanche avec la surface intérieure (24) du cylindre (12).

5. Appareil selon la revendication 1 ou 2, dans lequel la membrane perméable aux gaz et imperméable aux liquides (48) est formée à partir d'un matériau comprenant au moins l'un du polytétrafluoréthylène, du polypropylène et du polyéthylène.

6. Appareil selon la revendication 1, dans lequel le corps (50) a une pluralité de passages s'étendant à travers le corps de la première extrémité (52) à la seconde extrémité (54), et dans lequel l'élément filtrant (16) a en outre une pluralité de membranes perméables aux gaz et imperméables aux liquides, au moins l'une des membranes perméables aux gaz et imperméables aux liquides s'étendant à travers chacun des passages du corps (50).

7. Appareil selon la revendication 6, dans lequel l'élément filtrant (16) comprend en outre une pluralité de matériaux filtrants poreux positionnés entre la première extrémité du corps (50) et chacune des membranes perméables aux gaz et imperméables aux liquides pour empêcher les particules solides d'entrer en contact avec les membranes perméables aux gaz et imperméables aux liquides.

8. Appareil selon la revendication 6, dans lequel la pluralité de membranes perméables aux gaz et imperméables aux liquides (48) sont fixées au corps (50) de façon adjacente à la seconde extrémité (54) du corps.

9. Appareil selon la revendication 6, dans lequel les passages sont en relation parallèle les uns par rapport aux autres.

10. Procédé d'élimination de bulles d'un échantillon de fluide (32) ayant une partie liquide et une partie gazeuse à l'aide d'un appareil (10) selon la revendication 1, le procédé comprenant :
la réception d'un flux de l'échantillon de fluide (32) depuis une ouverture d'entrée (28) d'un cylindre (12) ayant une première extrémité (18), une seconde extrémité (20), une paroi latérale (22) s'étendant entre la première extrémité et la seconde extrémité, et une surface intérieure (24) définissant une chambre interne (26), la première extrémité (18) ayant l'ouverture d'entrée (28) et la seconde extrémité ayant une ouverture de sortie (30) ;
le passage de l'échantillon de fluide dans la chambre interne par le biais de l'ouverture d'entrée (28) ;
la mise en contact de l'échantillon de fluide (32) avec un élément filtrant (16) disposé à l'intérieur de la chambre interne (26), l'élément filtrant (16) définissant un côté entrée (44) et un côté sortie (46) de la chambre interne, l'élément filtrant (16) comportant une membrane (48) perméable aux gaz et imperméable aux liquides ;
la séparation d'au moins une partie de la partie gazeuse de l'échantillon liquide de la partie liquide de l'échantillon liquide en mettant l'échantillon liquide (32) en contact avec la membrane (48) perméable aux gaz et imperméable aux liquides de telle sorte qu'au moins une partie de la partie gazeuse de l'échantillon de fluide (32) passe à travers l'élément filtrant (16) du côté entrée au côté sortie (46) de la chambre interne (26) et que la partie liquide de l'échantillon de fluide (32) est empêchée de passer du côté entrée (44) au côté sortie (46) ;
la collecte d'au moins une partie de la partie liquide de l'échantillon de fluide (32) depuis le côté entrée de la chambre interne (26).

11. Procédé selon la revendication 10, dans lequel au moins une partie de la partie liquide de l'échantillon de fluide (32) est collectée depuis le côté entrée (44) de la chambre interne en perçant l'élément de filtre de telle sorte qu'une sonde peut être passée à travers l'élément filtrant (16) du côté sortie (46) au côté entrée (44) pour retirer la partie liquide de l'échantillon de fluide depuis le côté entrée (44) de la chambre interne (26).

12. Procédé selon la revendication 10, dans lequel l'élément filtrant (16) comprend en outre un élément filtrant interposé entre la membrane perméable aux gaz et imperméable aux liquides et le côté entrée (44) de la chambre interne (26), l'au moins un élément filtrant ayant un orifice d'entrée et un orifice de sortie, l'orifice d'entrée étant en communication fluidique avec le côté entrée (44) de la chambre interne (26), l'orifice de sortie étant en communication fluidique avec la membrane perméable aux gaz et imperméable aux liquides pour permettre à au moins une partie de l'échantillon de fluide (32) de passer à travers le ou les éléments filtrants et vers la membrane perméable aux gaz et imperméable aux liquides.

13. Procédé selon la revendication 10, dans lequel la membrane perméable aux gaz et imperméable aux liquides est constituée d'une pluralité de membranes perméables aux gaz et imperméables aux liquides.
